# EUROPEAN PATENT SPECIFICATION

(11) **EP 1 711 485 B1**
(45) Date of publication and mention of the grant of the patent: **13.05.2009**
(21) Application number: 04804953.0
(22) Date of filing: 20.12.2004
(51) Int. Cl.: C07D 401/06, A61K 31/4184, C07D 401/14, C07D 405/14, A61P 11/00, A61P 31/12

(54) **AMINO-BENZIMIDAZOLES DERIVATIVES AS INHIBITORS OF RESPIRATORY SYNCYTIAL VIRUS REPLICATION**
AMINOBENZIMIDAZOLDERIVATE ALS INHIBITOREN DER REPLIKATION DES RESPIRATORY SYNCYTIAL VIRUS
DERIVES DE AMINO-BENZIMIDAZOLE COMME INHIBITEURS DE REPLICATION DU VIRUS RESPIRATOIRE SYNCYTIAL

(30) Priority: 18.12.2003 EP 03104804; 30.04.2004 US 567181 P
(43) Date of publication of application: 18.10.2006
(73) Proprietor: Tibotec Pharmaceuticals Ltd., Eastgate Little Island Co Cork (IE)
(72) Inventor: BONFANTI, Jean-François, F-27430 Andé (FR); ANDRIES, Koenraad, Jozef, Lodewijk, B-2340 Beerse (BE); JANSSENS, Frans, Eduard, B-2820 Bonheiden (BE); SOMMEN, François, Maria, B-2323 Wortel (BE); GUILLEMONT, Jerôme, Emile, Georges, F-27430 Andé (FR); LACRAMPE, Jean, Fernand, Armand, F-76240 Le Mesnil-Esnard (FR)
(74) Representative: Wante, Dirk Paul Maria
(86) International application number: PCT/EP2004/053617
(87) International publication number: WO 2005/058870

(56) References cited:
- WO-A-01/00611
- WO-A-01/00612
- WO-A-01/00615
- FR-A- 2 731 707
- EUR. J. MED. CHEM., vol. 27, no. 4, 1992, pages 395-400, XP009029532
- DA SETTIMO ANTONIO ET AL: "Synthesis of 2-methylaminobenzimidazole derivatives tested for antiinflammatory activity" IL FARMACO, ROME, IT, vol. 49, no. 12, 1994, pages 829-834, XP002954490 ISSN: 0014-827X
- DATABASE CA [Online] CHEMICAL ABSTRACTS SERVICE, COLUMBUS, OHIO, US; KOVALEV, G. V. ET AL: "Effects of condensed derivatives of benzimidazole on gastric secretion" XP002321510 retrieved from STN Database accession no. 1990:210542 & KHIMIKO-FARMATSEVTICHESKII ZHURNAL , 24(2), 127-30 CODEN: KHFZAN; ISSN: 0023-1134, 1990,
- DATABASE CA [Online] CHEMICAL ABSTRACTS SERVICE, COLUMBUS, OHIO, US; HUNGER, A. ET AL: "Benzimidazole and related heterocycles. VII. New 2-aminobenzimidazoles" XP002321511 retrieved from STN Database accession no. 1962:12985 & HELVETICA CHIMICA ACTA , 44, 1273-82 CODEN: HCACAV; ISSN: 0018-019X, 1961,
- DATABASE BEILSTEIN XP002321512 & ANISIMOVA ET AL.: KHIM. GETEROTSIKL. SOEDIN., vol. 23, no. 1, 1987, pages 59-63,
- DATABASE BEILSTEIN XP002321513 & SIMONOV ET AL.: KHIM. GETEROTSIKL. SOEDIN., vol. 5, 1969, page 184,
- DATABASE BEILSTEIN XP002321514 & ZVEZDINA ET AL.: KHIM. GETEROTSIKL. SOEDIN., vol. 6, 1970, page 419,
- MOLINA ET AL.: CHEMISCHE BERICHTE, vol. 127, no. 9, 1994, pages 1641-52, XP009045299

## Description

The present invention is concerned with amino-benzimidazole derivatives having antiviral activity, in particular, having an inhibitory activity on the replication of the respiratory syncytial virus (RSV). It further concerns their preparation and compositions comprising them, as well as their use as a medicine.

Human RSV or Respiratory Syncytial Virus is a large RNA virus, member of the family of Paramyxoviridae, subfamily pneumoviridae together with bovine RSV virus. Human RSV is responsible for a spectrum of respiratory tract diseases in people of all ages throughout the world. It is the major cause of lower respiratory tract illness during infancy and childhood. Over half of all infants encounter RSV in their first year of life, and almost all within their first two years. The infection in young children can cause lung damage that persists for years and may contribute to chronic lung disease in later life (chronic wheezing, asthma). Older children and adults often suffer from a (bad) common cold upon RSV infection. In old age, susceptibility again increases, and RSV has been implicated in a number of outbreaks of pneumonia in the aged resulting in significant mortality.

Infection with a virus from a given subgroup does not protect against a subsequent infection with an RSV isolate from the same subgroup in the following winter season. Re-infection with RSV is thus common, despite the existence of only two subtypes, A and B.

Today only three drugs have been approved for use against RSV infection. A first one is ribavirin, a nucleoside analogue, provides an aerosol treatment for serious RSV infection in hospitalized children. The aerosol route of administration, the toxicity (risk of teratogenicity), the cost and the highly variable efficacy limit its use. The other two drugs, RespiGam^{®} and palivizumab, polyclonal and monoclonal antibody immunostimulants, are intended to be used in a preventive way.

Other attempts to develop a safe and effective RSV vaccine have all met with failure thus far. Inactivated vaccines failed to protect against disease, and in fact in some cases enhanced disease during subsequent infection. Life attenuated vaccines have been tried with limited success. Clearly there is a need for an efficacious non-toxic and easy to administer drug against RSV replication.

Benzimidazoles and imidazopyridines as inhibitors of RSV replication have been described in WO 01/00611, WO 01/00612 and WO 01/00615.

The present invention concerns inhibitors of RSV replication, which can be represented by formula (I): 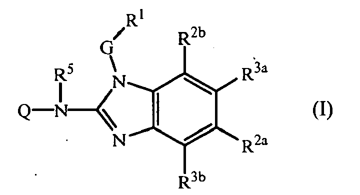 their *N*-oxides, addition salts, quaternary amines, metal complexes and stereochemically isomeric forms; wherein
- Q: is C₁₋₆alkyl substituted with one or two hydroxy substituents;
- G: is methylene;
- R¹: is pyridyl substituted with 1 or 2 substituents selected from the group consisting of hydroxy and C₁₋₆alkyl
- one of R^{2a} and R^{3a}: is C₁₋₆alkyl and the other one of R^{2a} and R^{3a} is hydrogen;
- in case R^{2a}: is different from hydrogen then R^{2b} is hydrogen or C₁₋₆alkyl, and R^{3b} is hydrogen;
- in case R^{3a}: is different from hydrogen then R^{3b} is hydrogen or C₁₋₆alkyl, and R^{2b} is hydrogen; or
- R^{2a}, R^{2b}, R^{3a} and R^{3b}: all are hydrogen;
- R⁵: is hydrogen or C₁₋₆alkyl.

The invention relates to the use of a compound of formula (I), or a *N*-oxide, addition salt, quaternary amine, metal complex and stereochemically isomeric form thereof, for the manufacture of a medicament for inhibiting RSV replication.

In a further aspect, this invention relates to methods for preparing these compounds.

A hydroxyC₁₋₆alkyl group when substituted on a nitrogen atom preferably is a hydroxyC₂₋₆akyl group wherein the hydroxy group and the oxygen or nitrogen is separated by at least two carbon atoms.

As used herein C₁₋₃alkyl as a group or part of a group defines straight or branched chain saturated hydrocarbon radicals having from 1 to 3 carbon atoms such as methyl, ethyl, propyl, 1-methylethyl and the like; C₁₋₄alkyl as a group or part of a group defines straight or branched chain saturated hydrocarbon radicals having from 1 to 4 carbon atoms such as the group defined for C₁₋₃alkyl and butyl and the like; C₂₋₄alkyl as a group or part of a group defines straight or branched chain saturated hydrocarbon radicals having from 2 to 4 carbon atoms such as ethyl, propyl, 1-methylethyl, butyl and the like; C₁₋₅alkyl as a group or part of a group defines straight or branched chain saturated hydrocarbon radicals having from 1 to 5 carbon atoms such as the groups defined for C₁₋₄alkyl and pentyl, 1-methylbutyl, 2-methylbutyl, 1-ethylpropyl and the like; C₁₋₆alkyl as a group or part of a group defines straight or branched chain saturated hydrocarbon radicals having from 1 to 6 carbon atoms such as the groups defined for C₁₋₅alkyl and hexyl, 2-methylpentyl, 3-methylpentyl and the like.

C₃₋₇cycloalkyl is generic to cyclopropyl, cyclobutyl, cyclopentyl, cyclohexyl and cycloheptyl.

As used herein before, the term (=O) forms a carbonyl moiety when attached to a carbon atom, a sulfoxide moiety when attached to a sulfur atom and a sulfonyl moiety when two of said terms are attached to a sulfur atom.

The term halo is generic to fluoro, chloro, bromo and iodo.

It should be noted that the radical positions on any molecular moiety used in the definitions may be anywhere on such moiety as long as it is chemically stable.

Radicals used in the definitions of the variables include all possible isomers unless otherwise indicated. For instance pyridyl includes 2-pyridyl, 3-pyridyl and 4-pyridyl; pentyl includes 1-pentyl, 2-pentyl and 3-pentyl.

When any variable occurs more than one time in any constituent, each definition is independent.

Whenever used hereinafter, the term 'compounds of formula (I)', or 'the present compounds' or similar term is meant to include the compounds of general formula (I), their *N*-oxides, addition salts, quaternary amines, metal complexes and stereochemically isomeric forms. An interesting subgroup of the compounds of formula (I) or any subgroup thereof are the *N*-oxides, salts and all the stereoisomeric forms of the compounds of formula (I).

It will be appreciated that some of the compounds of formula (I) may contain one or more centers of chirality and exist as stereochemically isomeric forms.

The term "stereochemically isomeric forms" as used hereinbefore defines all the possible compounds made up of the same atoms bonded by the same sequence of bonds but having different three-dimensional structures which are not interchangeable, which the compounds of formula (I) may possess.

Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of the present invention both in pure form or in admixture with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term 'stereoisomerically pure' concerns compounds or intermediates having a stereoisomeric excess of at least 80% (i. e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms 'enantiomerically pure' and 'diastereomerically pure' should be understood in a similar way, but then having regard to the enantiomeric excess, respectively the diastereomeric excess of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphosulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of formula (I) can be obtained separately by conventional methods. Appropriate physical separation methods that may advantageously be employed are, for example, selective crystallization and chromatography, e.g. column chromatography.

For some of the compounds of formula (I), their N-oxides, salts, solvates, quaternary amines, or metal complexes and the intermediates used in the preparation thereof, the absolute stereochemical configuration was not experimentally determined. A person skilled in the art is able to determine the absolute configuration of such compounds using art-known methods such as, for example, X-ray diffraction.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

For therapeutic use, salts of the compounds of formula (I) are those wherein the counterion is pharmaceutically acceptable. However, salts of acids and bases, which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove are meant to comprise the therapeutically active non-toxic acid and base addition salt forms which the compounds of formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butane-dioic acid), maleic, fumaric, malic (i.e. hydroxybutanedioic acid), tartaric, citric, methanesulfonic, ethanesulfonic, benzenesulfonic, *p*-toluenesulfonic, cyclamic, salicylic, *p*-aminosalicylic, pamoic and the like acids.

Conversely said salt forms can be converted by treatment with an appropriate base into the free base form.

The compounds of formula (I) containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term addition salt as used hereinabove also comprises the solvates which the compounds of formula (I) as well as the salts thereof, are able to form. Such solvates are for example hydrates, alcoholates and the like.

The term "quaternary amine" as used hereinbefore defines the quaternary ammonium salts which the compounds of formula (I) are able to form by reaction between a basic nitrogen of a compound of formula (I) and an appropriate quaternizing agent, such as, for example, an optionally substituted alkyl halide, aryl halide or arylalkyl halide, e.g. methyl iodide or benzyl iodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The *N*-oxide forms of the present compounds are meant to comprise the compounds of formula (I) wherein one or several nitrogen atoms are oxidized to the so-called *N*-oxide.

It will be appreciated that the compounds of formula (I) may have metal binding, chelating, complex forming properties and therefore may exist as metal complexes or metal chelates. Such metalated derivatives of the compounds of formula (I) are intended to be included within the scope of the present invention.

Some of the compounds of formula (I) may also exist in their tautomeric form. Such forms although not explicitly indicated in the above formula are intended to be included within the scope of the present invention.

Any subgroup of compounds of formula (I) specified herein is meant to also comprise the *N*-oxides, addition salts, quaternary amines, metal complexes and stereochemically isomeric forms of this subgroup of compounds of formula (I).

One embodiment of the present invention concerns compounds of formula (I-a): 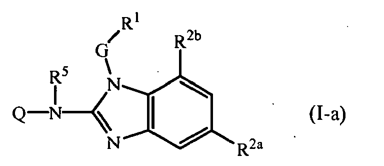
wherein Q, R⁵, G and R¹ are as specified above or as in any of the subgroups of compounds specified herein; and
R^{2a} is C₁₋₆alkyl;
R^{2b} is hydrogen or C₁₋₆alkyl.

Another embodiment of the present invention concerns compounds of formula (I-b): 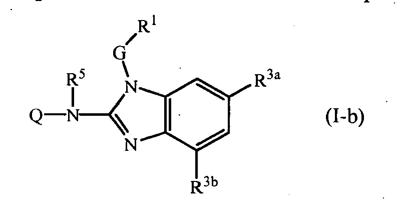
wherein Q, R⁵, G and R¹ are as specified above or as in any of the subgroups of compounds specified herein; and
R^{3a} is C₁₋₆alkyl;
R^{3b} is hydrogen or C₁₋₆alkyl.

Another embodiment of the present invention concerns compounds of formula (I-c): 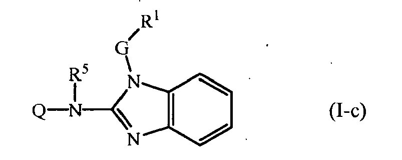 wherein Q, R⁵, G and R¹ are as specified above or as in any of the subgroups of compounds specified herein.

It is to be understood that the above defined subgroups of compounds of formulae (I-a), (I-b), etc. as well as any other subgroup defined herein, are meant to also comprise any *N*-oxides, addition salts, quaternary amines, metal complexes and stereochemically isomeric forms of such compounds.

Further particular subgroups of the compounds of formula (I) are those compounds of formula (I), or any subgroup of compounds of formula (I) specified herein, wherein
(a) R¹ is pyridyl substituted with hydroxy and C₁₋₆alkyl; or more preferably wherein
(b) R¹ is pyridyl substituted with hydroxy and methyl; or wherein
(c) R¹ is 3-hydroxy-6-methylpyrid-2-yl.

Preferred subgroups of compounds of formula (I) or any of the subgroups of compounds of formula (I) are those wherein G is methylene and R¹ is as specified above in (a) - (c).

Other embodiments comprise those compounds of formula (I) or any of the subgroups of compounds of formula (I) specified herein, wherein R⁵ is hydrogen.

Further particular subgroups of the compounds of formula (I) are those compounds of formula (I), or any subgroup of compounds of formula (I) specified herein, wherein one of R^{2a} and R^{3a} is C₁₋₆alkyl and the other one of R^{2a} and R^{3a} is hydrogen;
in case R^{2a} is different from hydrogen then R^{2b} is C₁₋₆alkyl, and R^{3b} is hydrogen;
in case R^{3a} is different from hydrogen then R^{3b} is C₁₋₆alkyl, and R^{2b} is hydrogen.

Preferred compounds are those compounds listed in tables 1 and 2.

The compounds of formula (I) or any of the subgroups thereof can be prepared as in the following reaction schemes.

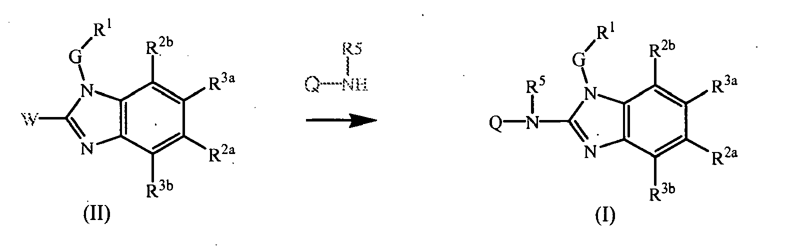

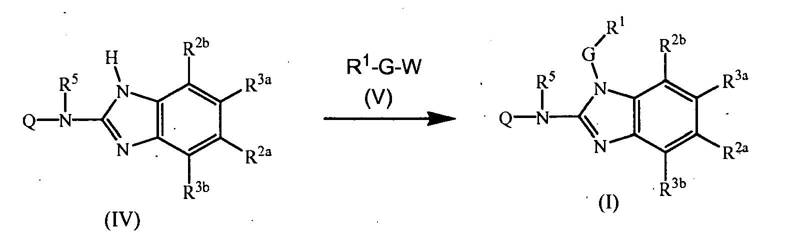

In these schemes Q, G, R¹, R^{2a}, R^{2b}, R^{3a}, R^{3b}, R⁵ have the meanings defined above for the compounds of formula (I) or of any of the subgroups thereof. W is an appropriate leaving group, preferably it is chloro or bromo. The reactions of these schemes can be typically conducted in a suitable solvent such as an ether, e.g. THF, a halogenated hydrocarbon, e.g. dichoromethane, CHCl₃, toluene, a polar aprotic solvent such as DMF, DMSO, DMA and the like. A base may be added to pick up the acid that is liberated during the reaction. If desired, certain catalysts such as iodide salts (e.g. KI) may be added.

Compounds of formula (I) may be converted into each other following art-known functional group transformation reactions, comprising those described hereinafter. Compounds of formula (I) wherein R⁵ is hydrogen may be converted to corresponding compounds of formula (I) wherein is other than hydrogen by an N-alkylation reaction which may be conducted under similar conditions as described above for the conversion of (II) or (IV) to (I).

Compounds wherein Q is C₁₋₆alkyl substituted with C₁₋₄alkoxycarbonyl can be reduced with e.g. LiAlH₄ to the corresponding compounds wherein Q is C₁₋₆alkyl substituted with hydroxy. The same starting materials can be reacted with an amine to the corresponding amides.

Compounds of formula (I) wherein Q is Ar having a cyano or a cyanoC₁₋₅alkyl substituent can be reduced with hydrogen in the presence of a suitable catalyst such as Raney nickel, to yield the corresponding methyleneamine or aminoC₁₋₆alkyl substituents.

A number of the intermediates used to prepare the compounds of formula (I) are known compounds or are analogs of known compounds, which can be prepared following modifications of art-known methodologies readily accessible to the skilled person. A number of preparations of intermediates are given hereafter in somewhat more detail. The intermediates of formula (II) and (IV) can be prepared as outlined in the following reaction schemes.

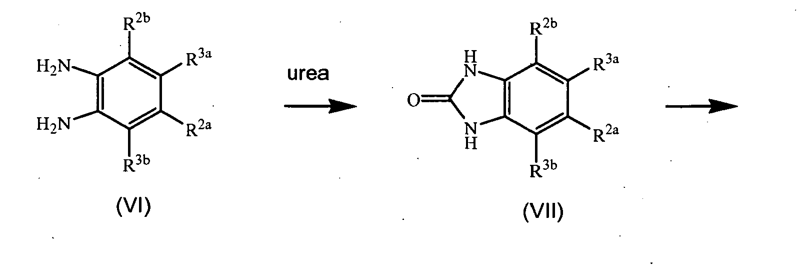

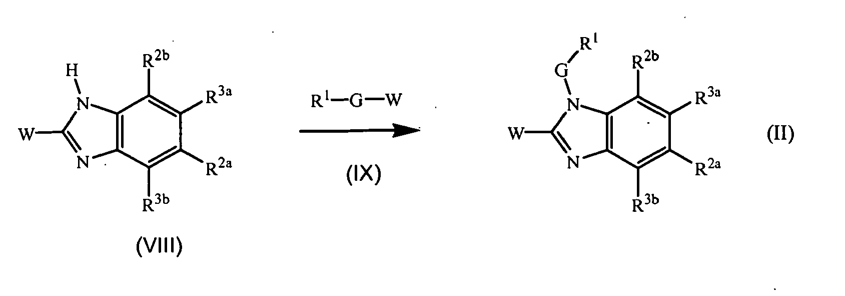

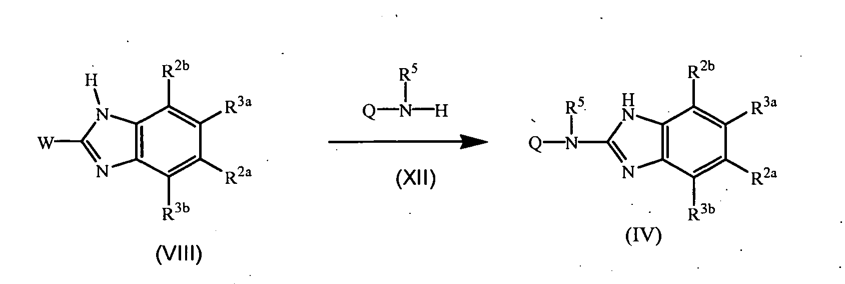

In a first step, a diaminobenzene (VI) is cyclized with urea, preferably in a suitable solvent, e.g. xylene, to yield a benzimidazolone (VII). The latter is converted to a benzimidazole derivative (VIII) wherein W is a leaving group as specified above, in particular by reaction of (VII) with a suitable halogenating agent, for example POCl₃. The resulting intermediate (VIII) is reacted with an amine derivative (IX) in an N-alkylation reaction to obtain an intermediate (II).

Intermediate (VIII) can be similarly reacted with an amine (XII) to yield intermediate (IV). The above-mentioned reactions are conducted in a suitable solvent and, if desired, in the presence of a base.

The compounds of formula (I) may be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Pure stereochemically isomeric forms of the compounds of formula (I) may be obtained by the application of art-known procedures. Diastereomers may be separated by physical methods such as selective crystallization and chromatographic techniques, e.g., countercurrent distribution, liquid chromatography and the like.

The compounds of formula (I) as prepared in the hereinabove described processes are generally racemic mixtures of enantiomers which can be separated from one another following art-known resolution procedures. The racemic compounds of formula (I) which are sufficiently basic or acidic may be converted into the corresponding diastereomeric salt forms by reaction with a suitable chiral acid, respectively chiral base. Said diastereomeric salt forms are subsequently separated, for example, by selective or fractional crystallization and the enantiomers are liberated therefrom by alkali or acid. An alternative manner of separating the enantiomeric forms of the compounds of formula (I) involves liquid chromatography, in particular liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

In a further aspect, the present invention concerns a pharmaceutical composition comprising a therapeutically effective amount of a compound of formula (I) as specified herein, or a compound of any of the subgroups of compounds of formula (I) as specified herein, and a pharmaceutically acceptable carrier. A therapeutically effective amount in this context is an amount sufficient to prophylaxictically act against, to stabilize or to reduce viral infection, and in particular RSV viral infection, in infected subjects or subjects being at risk of being infected. In still a further aspect, this invention relates to a process of preparing a pharmaceutical composition as specified herein, which comprises intimately mixing a pharmaceutically acceptable carrier with a therapeutically effective amount of a compound of formula (I), as specified herein, or of a compound of any of the subgroups of compounds of formula (I) as specified herein.

Therefore, the compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media maybe employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

The compounds of the present invention may also be administered via oral inhalation or insufflation by means of methods and formulations employed in the art for administration via this way. Thus, in general the compounds of the present invention may be administered to the lungs in the form of a solution, a suspension or a dry powder, a solution being preferred. Any system developed for the delivery of solutions, suspensions or dry powders via oral inhalation or insufflation are suitable for the administration of the present compounds.

Thus, the present invention also provides a pharmaceutical composition adapted for administration by inhalation or insufflation through the mouth comprising a compound of formula (I) and a pharmaceutically acceptable carrier. Preferably, the compounds of the present invention are administered via inhalation of a solution in nebulized or aerosolized doses.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compounds of formula (I) show antiviral properties. Viral infections treatable using the compounds and methods of the present invention include those infections brought on by ortho- and paramyxoviruses and in particular by human and bovine respiratory syncytial virus (RSV): A number of the compounds of this invention moreover are active against mutated strains of RSV. Additionally, many of the compounds of this invention show a favorable pharmacokinetic profile and have attractive properties in terms of bioavailabilty, including an acceptable half-life, AUC and peak values and lacking unfavourable phenomena such as insufficient quick onset and tissue retention.

The *in vitro* antiviral activity against RSV of the present compounds was tested in a test as described in the experimental part of the description, and may also be demonstrated in a virus yield reduction assay. The *in vivo* antiviral activity against RSV of the present compounds may be demonstrated in a test model using cotton rats as described in Wyde et al. (Antiviral Research (1998), 38, 31-42).

Due to their antiviral properties, particularly their anti-RSV properties, the compounds of formula (I) or any subgroup thereof, their *N*-oxides, addition salts, quaternary amines, metal complexes and stereochemically isomeric forms, are useful in the treatment of individuals experiencing a viral infection, particularly a RSV infection, and for the prophylaxis of these infections. In general, the compounds of the present invention may be useful in the treatment of warm-blooded animals infected with viruses, in particular the respiratory syncytial virus.

The compounds of the present invention or any subgroup thereof may therefore be used as medicines. Said use as a medicine or method of treatment comprises the systemic administration to viral infected subjects or to subjects susceptible to viral infections of an amount effective to combat the conditions associated with the viral infection, in particular the RSV infection.

The present invention also relates to the use of the present compounds or any subgroup thereof in the manufacture of a medicament for the treatment or the prevention of viral infections, particularly RSV infection.

In general it is contemplated that an antiviral effective daily amount would be from 0.01 mg/kg to 500 mg/kg body weight, more preferably from 0.1 mg/kg to 50 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

Also, the combination of another antiviral agent and a compound of formula (I) can be used as a medicine. Thus, the present invention also relates to a product containing (a) a compound of formula (I), and (b) another antiviral compound, as a combined preparation for simultaneous, separate or sequential use in antiviral treatment. The different drugs may be combined in a single preparation together with pharmaceutically acceptable carriers. For instance, the compounds of the present invention may be combined with interferon-beta or tumor necrosis factor-alpha in order to treat or prevent RSV infections.

### Examples

The following examples are intended to illustrate the present invention and not to limit it thereto. The terms 'compound 1, compound 4, etc. used in these examples refers to the same compounds in the tables.

The compounds were identified by LC/MS using the following equipment:
LCT: electrospray ionisation in positive mode, scanning mode from 100 to 900 amu; Xterra MS C18 (Waters, Milford, MA) 5 µm, 3.9 x 150 mm); flow rate 1 ml/min. Two mobile phases (mobile phase A: 85% 6.5mM ammonium acetate + 15% acetonitrile; mobile phase B: 20% 6.5 mM ammonium acetate + 80% acetonitrile) were employed to run a gradient from 100 % A for 3 min to 100% B in 5 min., 100% B for 6 min to 100 % A in 3 min, and equilibrate again with 100 % A for 3 min).
ZQ: electrospray ionisation in both positive and negative (pulsed) mode scanning from 100 to 1000 amu; Xterra RP C18 (Waters, Milford, MA) 5 µm, 3.9 x 150 mm); flow rate 1 ml/min. Two mobile phases (mobile phase A: 85% 6.5mM ammonium acetate + 15% acetonitrile; mobile phase B: 20% 6.5 mM ammonium acetate + 80% acetonitrile) were employed to run a gradient condition from 100 % A for 3 min to 100% B in 5 min., 100% B for 6 min to 100 % A in 3 min, and equilibrate again with 100 % A for 3 min).

### Example 1: Preparation of intermediate a-4.

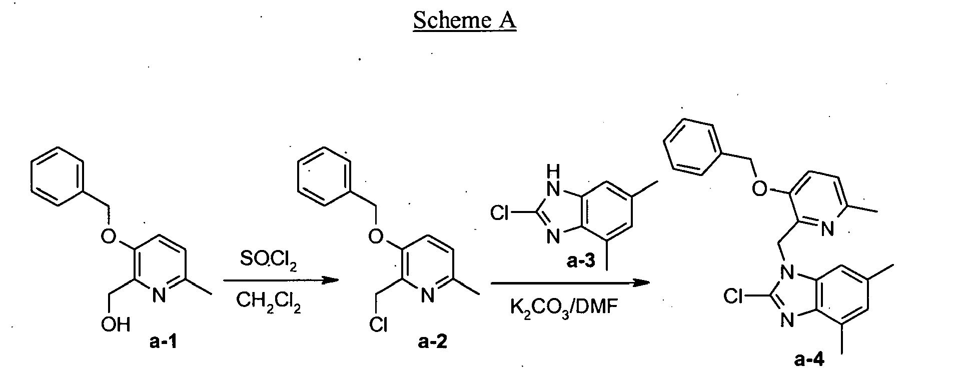

### Preparation of intermediate a-2:

SOCl₂ (14 ml) was added drop wise to a solution of (3-benzyloxy-6-methyl-pyridin-2-yl)-methanol (0.0606 mol) at 5°C. The reaction was stirred at room temperature for 3 hours. The solvent was evaporated under reduced pressure. The residue was taken up in diethyl ether. The precipitate was filtered off and dried, yielding 16.9g of **a-2** (98%, melting point: 182°C).

### Preparation of intermediate a-4:

A mixture of 2-chloro-4,6-dimethyl-1H-benzimidazole (0.083 mol), **a-2** (0.0913 mol) and K₂CO₃ (0.332 mol) in dimethylformamide (100ml) was stirred at room temperature for 24 hours. H₂O was then added. The mixture was extracted three times with CH₂Cl₂. The organic layer was separated, dried (over MgSO₄), filtered and the solvent was evaporated at 30°C under reduced pressure. The residue was taken up in CH₃CN/ diisopropylether. The precipitate was filtered off and dried, yielding 16.8g of **a-4** (52%, melting point: 155°C).

### Example 2: Preparation of dihydroxyalkyl substituted dimethylbenzimidazoleamines

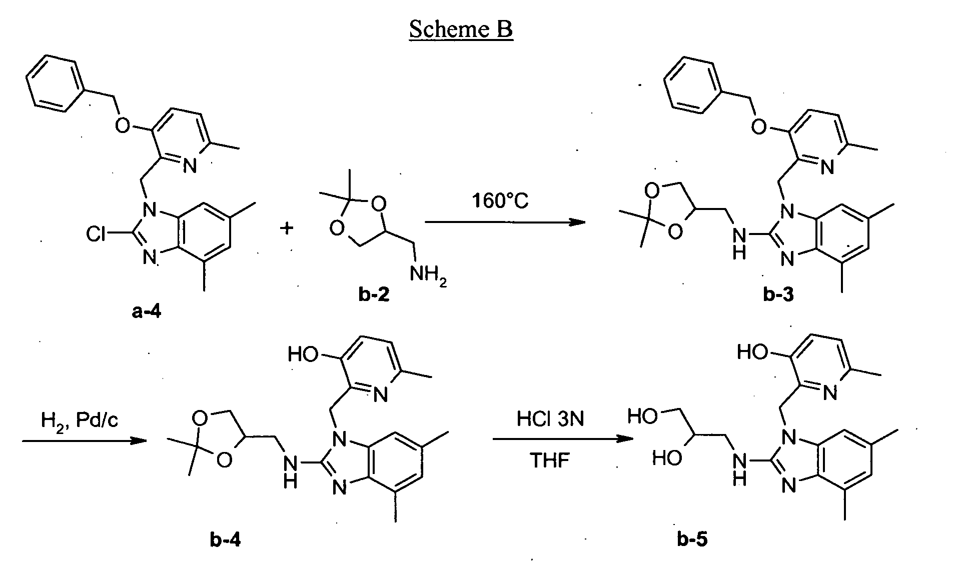

### Preparation of intermediate b-3:

A mixture of **a-4** (0.0014 mol) and **b-2** (0.0012 mol) was stirred at 130°C for 3 hours, then stirred at 160°C for 2 hours, cooled down to room temperature and taken up in CH₂Cl₂. The organic layer was washed with a 10% solution of K₂CO₃, dried (over MgSO₄), filtered and the solvent was evaporated until dryness. The residue was purified by column chromatography over silica gel (eluent: CH₂Cl₂/CH₃OH/NH₄OH 97/3/0.1). The pure fractions were collected and the solvent was evaporated, yielding 0.55g of intermediate **b-3** (81%).

### Preparation of compound b-4:

A mixture of **b-3** (0.0011 mol) and Pd/C (0.18g) in CH₃OH (10ml) was hydrogenated for 1 hour under a 3 bar pressure, then filtered over celite. Celite was rinsed with CH₃OH. The filtrate was concentrated under reduced pressure. The residue (0.47g) was crystallized from CH₃CN. The precipitate was filtered off and dried, yielding 0.27g of 2-{2-[(2,2-dimethyl-[1,3]dioxolan-4-ylmethyl)-amino]-4,6-dimethyl-benzoimidazol-1-ylmethyl}-6-methyl-pyridin-3-ol (compound 21, 60%, melting point: 225°C).

### Preparation of final compound b-5:

A mixture of 2-{2-[(2,2-dimethyl-[1,3]dioxolan-4-ylmethyl)-amino]-4,6-dimethyl-benzoimidazol-1-ylmethyl}-6-methyl-pyridin-3-ol (0.0005 mol) in a 3N solution of HCl (15ml) and tetrahydrofuran (15ml) was stirred for 4 hours. Tetrahydrofuran was evaporated under reduced pressure. The mixture was basified with K₂CO₃ (powder). The aqueous layer was saturated with K₂CO₃ (powder). A solution of CH₂Cl₂/CH₃OH (90/10) was added. The organic layer was separated, dried (over MgSO₄), filtered and the solvent was evaporated. The residue (0.17g, 88%) was crystallized from CH₃CN/ diisopropylether. The precipitate was filtered off and dried. Yielding: 0.085g of 3-[1-(3-hydroxy-6-methyl-pyridin-2-ylmethyl)-4,6-dimethyl-1H-benzoimidazol-2-ylamino]-propane-1,2-diol (compound 4, melting point: 205°C).

### Example 3: Preparation of hydroxyalkyl substituted dimethylbenzimidazoleamines

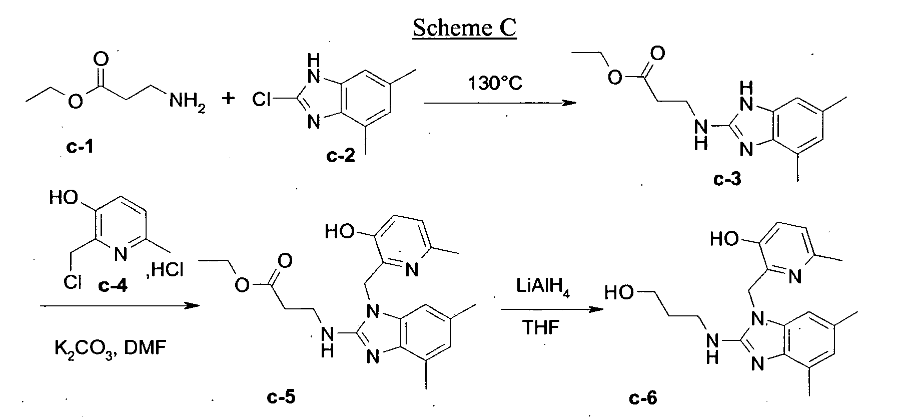

### Preparation of intermediate c-3:

A mixture of **c-2** (0.004 mol) and **c-1** (0.006 mol) was stirred at 130°C for 12 hours, and then taken up in CH₂Cl₂. The organic layer was washed with a 10% solution of K₂CO₃, dried (over MgSO₄), filtered and the solvent was evaporated until dryness. The residue (0.6g) was purified by column chromatography over silica gel (eluent: CH₂Cl₂/CH₃OH/NH₄OH 95/5/0.2; 10µm). The pure fractions were collected and the solvent was evaporated, yielding 0.4g of intermediate **c-3** (38%).

### Preparation of compound c-5:

A mixture of **c-3** (0.0015 mol), **c-4** (0.0016 mol) and K₂CO₃ (0.0052 mol) in dimethylformamide (20ml) was stirred at 70°C for 4 hours. The solvent was evaporated until dryness. The residue was taken up in CH₂Cl₂. The organic layer was washed with H₂O, dried (over MgSO₄), filtered and the solvent was evaporated. The residue (0.81 g) was purified by column chromatography over silica gel (eluent: toluene/2-propanol/NH₄OH 90/10/0.5; 10µm). The pure fractions were collected and the solvent was evaporated. The residue (0.12g) was crystallized from 2-propanol/CH₃CN/diisopropylether. The precipitate was filtered off and dried, yielding 0.12g of 3-[1-(3-hydroxy-6-methyl-pyridin-2-ylmethyl)-4,6-dimethyl-1H-benzoimidazol-2-ylamino]-propionic acid ethyl ester (compound 12, 21%, melting point: 180°C).

### Preparation of final compound c-6:

LiAlH₄ (0.0003 mol) was added portion wise at 5°C to a mixture of 3-[1-(3-hydroxy-6-methyl-pyridin-2-ylmethyl)-4,6-dimethyl-1H-benzoimidazol-2-ylamino]-propionic acid ethyl ester (0.0001 mol) in tetrahydrofuran (10ml) under N₂ flow. The mixture was stirred at 5°C for 1 hour, then at room temperature for 3 hours. Ethylacetate and H₂O were added. The mixture was extracted with ethylacetate. The organic layer was separated, dried (over MgSO₄), filtered and the solvent was evaporated until dryness. The residue was crystallized from 2-propanone/CH₃CN/ diisopropylether. The precipitate was filtered off and dried, yielding 0.025g of 2-[2-(3-hydroxypropylamino)-4,6-dimethyl-benzoimidazol-1-ylmethyl]-6-methyl-pyridin-3-ol (compound 7, 73%, melting point: 170°C).

The following tables list compounds of the present invention that were prepared analogous to any one of the above mentioned synthesis schemes.

**Table 1**

| | | | | | |
|---|---|---|---|---|---|
| 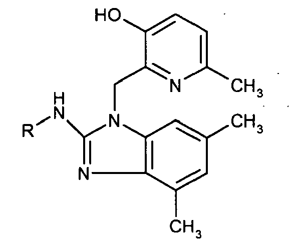 | | | | | |

| Comp. No. | R | Activity | Mass Spectroscopy | Melting point | Synthesis Scheme / salt form |
|---|---|---|---|---|---|
| b-5 | 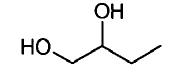 | *7.4* | MH⁺ = 357 | 205°C | B |
| c-6 | 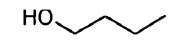 | *6.8* | MH⁺ = 341 | 170°C | C |

**Table 2**

| | | | | | |
|---|---|---|---|---|---|
| 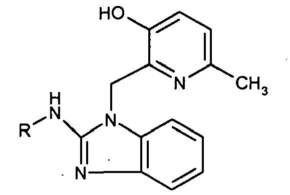 | | | | | |

| Comp. No. | R | Activity | Mass Spectroscopy | Melting point | Synthesis scheme |
|---|---|---|---|---|---|
| - | 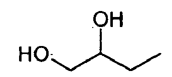 | *6.3* | MH⁺ = 329 | 170°C | C |

### Example 7: In vitro screening for activity against Respiratory Syncytial Virus.

The percent protection against cytopathology caused by viruses (antiviral activity or EC₅₀) achieved by tested compounds and their cytotoxicity (CC₅₀) are both calculated from dose-response curves. The selectivity of the antiviral effect is represented by the selectivity index (SI), calculated by dividing the CC₅₀ (cytotoxic dose for 50% of the cells) by the EC₅₀ (antiviral activity for 50 % of the cells). The tables in the above experimental part list the category to which each of the prepared compounds belong : Compounds belonging to activity category "A" have an pEC₅₀ (-log of EC₅₀ when expressed in molar units) equal to or more than 6. Compounds belonging to activity category "B" have a pEC50 value below 6.

Automated tetrazolium-based colorimetric assays were used for determination of EC₅₀ and CC₅₀ of test compounds. Flat-bottom, 96-well plastic microtiter trays were filled with 180 µl of Eagle's Basal Medium, supplemented with 5 % FCS (0% for FLU) and 20 mM Hepes buffer. Subsequently, stock solutions (7.8 x final test concentration) of compounds were added in 45 µl volumes to a series of triplicate wells so as to allow simultaneous evaluation of their effects on virus- and mock-infected cells. Five fivefold dilutions were made directly in the microtiter trays using a robot system. Untreated virus controls, and HeLa cell controls were included in each test. Approximately 100 TCID₅₀ of Respiratory Syncytial Virus was added to two of the three rows in a volume of 50 µl. The same volume of medium was added to the third row to measure the cytotoxicity of the compounds at the same concentrations as those used to measure the antiviral activity. After two hours of incubation, a suspension (4 x 10⁵ cells/ml) of HeLa cells was added to all wells in a volume of 50µl. The cultures were incubated at 37°C in a 5% CO₂ atmosphere. Seven days after infection the cytotoxicity and the antiviral activity was examined spectrophotometrically. To each well of the microtiter tray, 25 µl of a solution of MTT (3-(4,5-dimethylthiazol-2-yl)-2,5-diphenyltetrazolium bromide) was added. The trays were further incubated at 37°C for 2 hours, after which the medium was removed from each cup. Solubilization of the formazan crystals was achieved by adding 100 µl 2-propanol. Complete dissolution of the formazan crystals were obtained after the trays have been placed on a plate shaker for 10 min. Finally, the absorbances were read in an eight-channel computer-controlled photometer (Multiskan MCC, Flow Laboratories) at two wavelengths (540 and 690 nm). The absorbance measured at 690 nm was automatically subtracted from the absorbance at 540 nm, so as to eliminate the effects of non-specific absorption.

## Claims

1. A compound of formula (I) 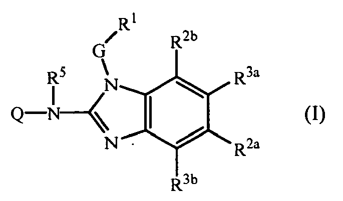 a *N*-oxide, addition salt, quaternary amine, metal complex or stereochemically isomeric form thereof, wherein
Q is C₁₋₆alkyl substituted with one or two hydroxy substituents;
G is methylene;
R¹ is pyridyl substituted with 1 or 2 substituents independently selected from the group consisting of hydroxy and C₁₋₆alkyl;
one of R^{2a} and R^{3a} is C₁₋₆alkyl and the other one of R^{2a} and R^{3a} is hydrogen;
in case R^{2a} is different from hydrogen then R^{2b} is hydrogen or C₁₋₆alkyl, and R^{3b} is hydrogen;
in case R^{3a} is different from hydrogen then R^{3b} is hydrogen or C₁₋₆alkyl, and R^{2b} is hydrogen; or
R^{2a}, R^{2b}, R^{3a} and R^{3b} all are hydrogen;
R⁵ is hydrogen or C₁₋₆alkyl.

2. A compound according to claim 1, wherein R¹ is pyridyl substituted with hydroxy and C₁₋₆alkyl.

3. A compound according to claim 1, wherein R¹ is pyridyl substituted with hydroxy and methyl.

4. A compound according to claim 1, wherein R¹ is 3-hydroxy-6-methylpyrid-2-yl.

5. A compound according to any of claims 1 - 4, wherein R⁵ is hydrogen.

6. A compound according to any of claims 1 - 5, wherein one of R^{2a} and R^{3a} is C₁₋₆alkyl and the other one of R^{2a} and R^{3a} is hydrogen;
in case R^{2a} is different from hydrogen then R^{2b} is C₁₋₆alkyl, and R^{3b} is hydrogen;
in case R^{3a} is different from hydrogen then R^{3b} is C₁₋₆alkyl, and R^{2b} is hydrogen.

7. A compound as claimed in any one of claims 1 to 6 for use as a medicine.

8. A pharmaceutical composition comprising a pharmaceutically acceptable carrier, and as active ingredient a therapeutically effective amount of a compound as claimed in any one of claims 1 to 6.

9. The use of a compound as claimed in any of claims 1 to 6 for the manufacture of a medicament for inhibiting RSV replication.

10. A process for preparing a compound as claimed in any of claims 1 to 6, said process comprising
(a) reacting an intermediate of formula (II) with a reagent (III) as in the following reaction scheme: 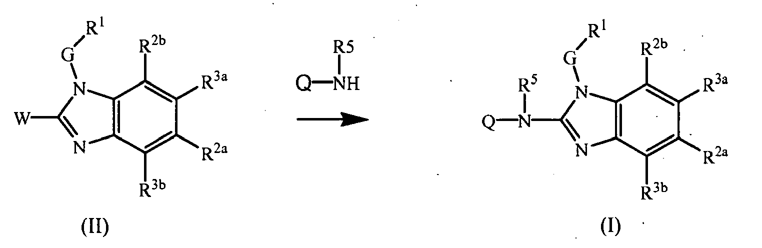
(b) reacting an intermediate of formula (IV) with a reagent (V) as in the following reaction scheme: 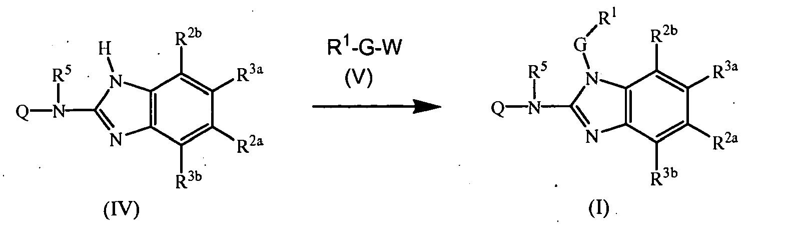
wherein Q, G, R¹, R^{2a}, R^{2b}, R^{3a}, R^{3b}, R⁵ are as claimed in any of claims 1 to 6 and W is a leaving group;
and optionally converting the thus obtained compounds of formula (I) into their pharmaceutically acceptable base-addition or acid addition salt form by treatment with a suitable base or acid and conversely treating the base-addition or acid addition salt form with an acid or a base to obtain the free form of the compound of formula (I).

## Patentansprüche

1. Verbindungen der Formel (I) 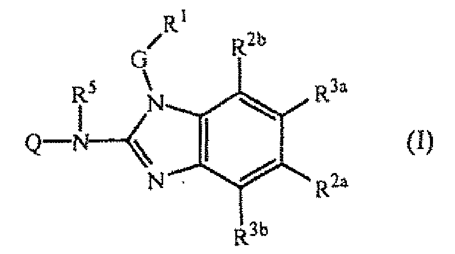
deren N-Oxide, Additionssalze, quaternäre Amine, Metallkomplexe und stereochemische isomere Formeln, wobei
Q für C₁₋₆-Alkyl, das mit einem oder zwei Hydroxysubstituenten substituiert ist, steht;
G für Methylen steht;
R¹ für Pyridyl, das mit 1 oder 2 unabhängig voreinander aus der Gruppe bestehend aus Hydroxy und C₁₋₆-Alkyl ausgewählten Substituenten substituiert ist, steht;
einer der Reste R^{2a} und R^{3a} für C₁₋₆-Alkyl steht und der andere der Reste R^{2a} und R^{3a} für Wasserstoff steht;
falls R^{2a} nicht für Wasserstoff steht, R^{2b} für Wasserstoff oder C₁₋₆-Alkyl steht und R^{3b} für Wasserstoff steht;
falls R^{3a} nicht für Wasserstoff steht, R^{3b} für Wasserstoff oder C₁₋₆-Alkyl steht und R^{2b} für Wasserstoff steht; oder
R^{2a}, R^{2b}, R^{3a} und R^{3b} alle für Wasserstoff stehen;
R⁵ für Wasserstoff oder C₁₋₆-Alkyl steht.

2. Verbindungen nach Anspruch 1, wobei R¹ für Pyridyl, das mit Hydroxy und C₁₋₆-Alkyl substituiert ist, steht.

3. Verbindungen nach Anspruch 1, wobei R¹ für Pyridyl, das mit Hydroxy und Methyl substituiert ist, steht.

4. Verbindungen nach Anspruch 1, wobei R¹ für 3-Hydroxy-6-methylpyrid-2-yl steht.

5. Verbindungen nach einem der Ansprüche 1 - 4, wobei R⁵ für Wasserstoff steht.

6. Verbindungen nach einem der Ansprüche 1 - 5, wobei einer der Reste R^{2a} und R^{3a} für C₁₋₆-Alkyl steht und der andere der Reste R^{2a} und R^{3a} für Wasserstoff steht;
falls R^{2a} nicht für Wasserstoff steht, R^{2b} für C₁₋₆-Alkyl steht und R^{3b} für Wasserstoff steht;
falls R^{3a} nicht für Wasserstoff steht, R^{3b} für C₁₋₆-Alkyl steht und R^{2b} für Wasserstoff steht.

7. Verbindungen nach einem der Ansprüche 1 bis 6 zur Verwendung als Medizin.

8. Pharmazeutische Zusammensetzung, enthaltend einen pharmazeutisch annehmbaren Träger und als Wirkstoff eine therapeutisch wirksame Menge einer Verbindung nach einem der Ansprüche 1 bis 6.

9. Verwendung einer Verbindung nach einem der Ansprüche 1 bis 6 zur Herstellung eines Medikaments zum Inhibieren der RSV-Replikation.

10. Verfahren zur Herstellung einer Verbindung nach einem der Ansprüche 1 bis 6, wobei das Verfahren folgendes umfaßt:
(a) die Umsetzung eines Zwischenprodukts der Formel (II) mit einem Reagens (III), wie in dem folgenden Reaktionsschema: 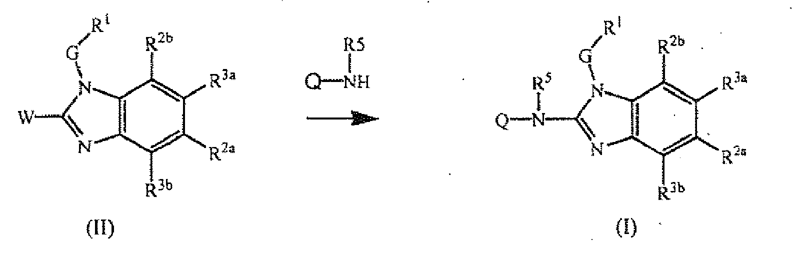
(b) die Umsetzung eines Zwischenprodukts der Formel (IV) mit einem Reagens (V), wie in dem folgenden Reaktionsschema: 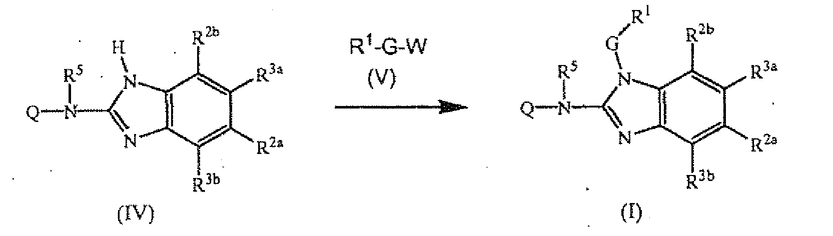 wobei Q, G, R¹, R^{2a}, R^{2b}, R^{3a}, R^{3b} und R⁵ wie in einem der Ansprüche 1 bis 6 definiert sind und W für eine Abgangsgruppe steht;
und gegebenenfalls die Umwandlung der auf diese Weise erhaltenen Verbindungen der Formel (I) in ihre pharmazeutisch annehmbare Basenadditions- oder Säureadditionssalzform durch Behandlung mit einer geeigneten Base bzw. Säure und umgekehrt die Behandlung der Basenadditions- oder Säureadditionssalzform mit einer Säure bzw. einer Base unter Erhalt der freien Form der Verbindung der Formel (I).

## Revendications

1. Composé de formule (I) 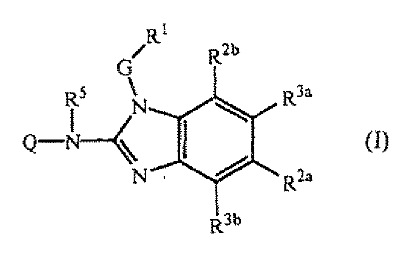
un N-oxyde, une sel d'addition, une amine quaternaire, un complexe métallique ou une forme stéréochimiquement isomère de celui-ci, dans laquelle
Q est un groupe alkyle en C₁₋₆ substitué par un ou deux substituants hydroxy ;
G est un groupe méthylène ;
R¹ est un groupe pyridyle substitué par 1 ou 2 substituants choisis indépendamment parmi le groupe constitué d'un groupe hydroxy et d'un groupe alkyle en C₁₋₆ ;
l'un parmi R^{2a} et R^{3a} est un groupe alkyle en C₁₋₆ et l'autre parmi R^{2a} et R^{3a} est un atome d' hydrogène ;
dans le cas où R^{2a} est différent d'un atome d'hydrogène, alors R^{2b} est un atome d'hydrogène ou un groupe alkyle en C₁₋₆, et R^{3b} est un atome d'hydrogène ;
dans le cas où R^{3a} est différent d'un atome d'hydrogène, alors R^{3b} est un atome d'hydrogène ou un groupe alkyle en C₁₋₆, et R^{2b} est un atome d'hydrogène ; ou
R^{2a}, R^{2b}, R^{3a} et R^{3b} sont tous un atome d'hydrogène ;
R⁵ est un atome d'hydrogène ou un groupe alkyle en C₁₋₆.

2. Composé selon la revendication 1, dans lequel R¹ est un groupe pyridyle substitué par un groupe hydroxy et un groupe alkyle en C₁₋₆.

3. Composé selon la revendication 1, dans lequel R¹ est un groupe pyridyle substitué par un groupe hydroxy et un groupe méthyle.

4. Composé selon la revendication 1, dans lequel R¹ est un groupe 3-hydroxy-6-méthylpyrid-2-yle.

5. Composé selon l'une quelconque des revendications 1 à 4, dans lequel R⁵ est un atome d'hydrogène.

6. Composé selon l'une quelconque des revendications 1 à 5, dans lequel l'un parmi R^{2a} et R^{3a} est un groupe alkyle en C₁₋₆ et l'autre parmi R^{2a} et R^{3a} est un atome d'hydrogène ;
dans le cas où R^{2a} est différent d'un atome d'hydrogène, alors R^{2b} est un groupe alkyle en C₁₋₆ et R^{3b} est un atome d'hydrogène ;
dans le cas où R^{3a} est différent d'un atome d'hydrogène, alors R^{3b} est un groupe alkyle en C₁₋₆ et R^{2b} est un atome d'hydrogène.

7. Composé selon l'une quelconque des revendications 1 à 6, destiné à être utilisé en tant que médicament.

8. Composition pharmaceutique comprenant un support pharmaceutiquement acceptable, et en tant qu'ingrédient actif, une quantité thérapeutiquement efficace d'un composé selon l'une quelconque des revendications 1 à 6.

9. Utilisation d'un composé selon l'une quelconque des revendications 1 à 6 pour la fabrication d'un médicament destiné à l'inhibition de la réplication du VRS.

10. Procédé de préparation d'un composé selon l'une quelconque des revendications 1 à 6, ledit procédé comprenant
a) la réaction d'un intermédiaire de formule (II) avec un réactif (III) comme dans le schéma réactionnel suivant : 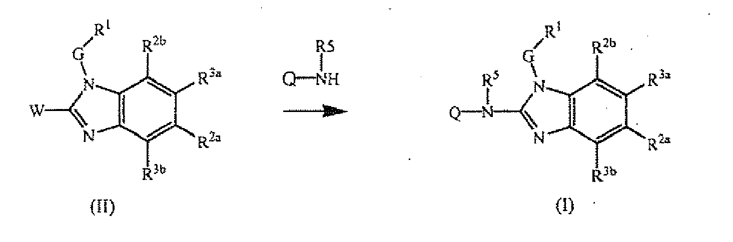
b) la réaction d'un intermédiaire de formule (IV) avec un réactif (V) comme dans le schéma réactionnel suivant : 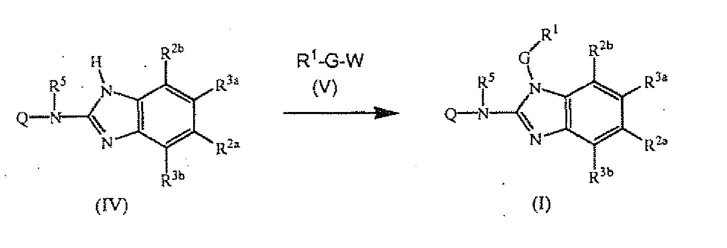
dans lesquels Q, G, R¹, R^{2a}, R^{2b}, R^{3a}, R^{3b}, R⁵ sont tels que revendiqués dans l'une quelconque des revendications 1 à 6 et W est un groupe partant,
et éventuellement la conversion des composés ainsi obtenus de formule (I) en leur forme de sel d'addition à une base ou d'addition à un acide, pharmaceutiquement acceptable, par traitement avec une base ou un acide approprié et, inversement le traitement de la forme de sel d'addition à une base ou d'addition à un acide avec un acide ou une base en vue d'obtenir la forme libre du composé de formule (I).
